(19) Europäisches Patentamt
European Patent Office
Office européen des brevets

(11) **EP 1 464 276 B1**

(12) **EUROPÄISCHE PATENTSCHRIFT**

(45) Veröffentlichungstag und Bekanntmachung des Hinweises auf die Patenterteilung:
**14.02.2007 Patentblatt 2007/07**

(51) Int Cl.:
*A61B 5/0275* *(2006.01)*    *A61B 5/026* *(2006.01)*

(21) Anmeldenummer: **04101074.5**

(22) Anmeldetag: **16.03.2004**

(54) **Vorrichtung zur Messung des Blutflusses in einem Organ**

Device for measuring blood flow in an organ

Dispositif de mesure d'écoulements sanguins dans un organe

(84) Benannte Vertragsstaaten:
**DE ES FR GB IT**

(30) Priorität: **05.04.2003 DE 10315574**

(43) Veröffentlichungstag der Anmeldung:
**06.10.2004 Patentblatt 2004/41**

(73) Patentinhaber:
• **Universität Zürich**
**8006 Zürich (CH)**
• **ETH Zürich**
**8092 Zürich (CH)**

(72) Erfinder:
• **Keller, Emanuela**
**8802, Kilchberg (CH)**
• **Nadler, Andreas**
**8820, Wädenswil (CH)**
• **Niederer, Peter**
**8037, Zürich (CH)**

(74) Vertreter: **Kehl, Günther**
**Kehl & Ettmayr Patentanwälte,**
**Friedrich-Herschel-Strasse 9**
**81679 München (DE)**

(56) Entgegenhaltungen:
EP-A- 0 615 723       US-A1- 2002 183 621
US-B1- 6 223 069       US-B1- 6 339 714

• **KELLER E. ET AL: "New Methods for Monitoring Cerebral Oxygenation and Hemodynamics in Patients with Subachnoid Hemorrhage" ACTA NEUROCHIRURGICA. SUPPLEMENT., Bd. 82, 2002, Seiten 87-92, XP008033028 AUSTRIA**
• **HOEFT A. ET AL.: "Bedside Assessment of Intravascular Volume Status in Patients Undergoing Coronary Bypass Surgery" ANESTHESIOLOGY, Bd. 81, Nr. 1, 1994, Seiten 76-86, XP008033021 HAGERSTOWN**

**Beschreibung**

Die vorliegende Erfindung betrifft eine Vorrichtung zur Messung des Blutflusses in einem Organ mit Hilfe eines injizierten Indikators.

Vorrichtungen zur Messung des Blutflusses in einem Organ, insbesondere des cerebralen Blutflusses (CBF), mit Hilfe eines injizierten, weitgehend inerten Indikators sind seit langem bekannt und teilweise auch im klinischen Einsatz. Auf älteren Verfahren, wie etwa der Xenon-Dilutionstechnik (Obrist, W. D., Thompson, H. K., Wang, H. S., and Wilkinson, W. E. 1975. *Regional cerebral blood flow estimated by 133-xenon inhalation.* Stroke 6: 245-256), basierende Vorrichtungen sind oft schwierig technisch umsetzbar und erweisen sich im Einsatz als zeitaufwendig. In jüngerer Zeit haben vor allem Technologien an Bedeutung gewonnen, welche die Nahinfrarotspektroskopie (NIRS) in Verbindung mit Indocyningrün (ICG) als Indikator einsetzen, um die cerebrale Durchblutung zu untersuchen bzw. zu überwachen.

In aller Regel erfordert der Einsatz derartiger Vorrichtungen die invasive Messung der Eingangsfunktion, d.h. der arteriellen Konzentration des Indikators über die Zeit. Dabei finden beispielsweise arterielle Faseroptikkatheter Verwendung.

In jüngster Zeit wurde auch die Anwendung nichtinvasiver Meßmethoden zur Überwachung der Gehirndurchblutung vorgeschlagen (Keller, E., Wolf, M., Martin, M., Fandino, J. and Yonekawa Y 2001. *Estimation of cerebral oxygenation and hemodynamics in cerebral vasospasm using indocyaningreen (ICG) dye dilution and near infrared spectroscopy (NIRS). A case report.* J. Neurosurg. Anesthesiol. 13: 43-48). Dabei werden Optoden am Kopf angebracht, welche als Sender bzw. Empfänger der nahinfraroten Strahlung fungieren, mittels welcher die Indikatorkonzentration im cerebralen Gefäßsystem bestimmt wird. Die zur Auswertung verwendeten Algorithmen sind in der Literatur beschrieben (Keller, E., Nadler, A., Imhof, H.-G., Niederer, P., Roth, P. and Yonekawa Y. 2002. *New Methods fo Monitoring Cerebral Oxygenation and Hemodynamics in Patients with Subarachnoid Hemorrhage.* Acta Neurochir. [Suppl.]82: 87-92).

Ein wesentliches Problem herkömmlicher NIRS-Vorrichtungen besteht darin, daß deren Auswerteeinheiten mit einer gewissen Ungenauigkeit arbeiten, welche darauf beruht, daß bei der Auswertung bisher von der vereinfachenden Annahme ausgegangen wird, während des ersten Anstiegs des Eingangssignals (der sogenannten "rise time") finde kein Abfluß des Indikators aus dem untersuchten System statt. Die Genauigkeit der im Rahmen der Auswertung gelieferten Ergebnisse hängt somit entscheidend von der Schärfe des ersten Peaks des Meßsignals nach der Indikatorgabe ab. Da sich aus einleuchtenden praktischen Gründen die Injektion des Indikators nicht in beliebig kurzer Zeit und nicht beliebig nah am Meßort realisieren läßt, war die Abhängigkeit der Qualität der Meßergebnisse von der Schärfe des ersten Peaks des Meßsignals nach der Indikatorgabe bisher als prinzipbedingt hinzunehmen.

Alternative Meßmethoden, wie etwa die Positronenemissionstomographie (PET), die Einzelphotonenemissionscomputertomographie (SPECT) oder die perfusionsgewichtete magnetische Resonanzspektroskopie, sind nur mittels äußerst kostspieliger Vorrichtungen technisch umsetzbar und erfordern im Einsatz den mitunter beträchtliche Risiken bergenden Transport des betreffenden Patienten zu der Meßeinheit; d.h. die entsprechenden Vorrichtungen lassen sich nicht als sogenannte "bedside"-Apparaturen am Patientenbett einsetzen.

Eine wesentliche Aufgabe der vorliegenden Erfindung besteht darin, eine Vorrichtung zu schaffen, mit welcher sich höhere Genauigkeiten bei der Bestimmung des Blutflusses in einem Organ, insbesondere des cerebralen Blutflusses, erzielen lassen. Insbesondere soll die Genauigkeit des Auswerteergebnisses weniger stark von den Randbedingungen, d.h. insbesondere von der Ausführung der Indikatorinjektion abhängen. Ferner soll die zu schaffende Vorrichtung nicht-invasiv einsetzbar und mit vertretbarem technischen Aufwand realisierbar sein.

Gemäß einem Aspekt der vorliegenden Erfindung wird diese Aufgabenstellung mit einer Vorrichtung nach Anspruch 1 gelöst. Vorteilhafte Ausgestaltungen der erfindungsgemäßen Vorrichtung sind in den Ansprüchen 2 bis 12 beschrieben.

Einer der Vorteile der vorliegenden Erfindung besteht darin, daß die Schärfe des ersten Peaks des Meßsignals nach Indikatorgabe - für den Fachmann völlig überraschend - nicht mehr entscheidend für die Genauigkeit der erzielbaren Ergebnisse ist, da die Auswerteeinheit aufgrund ihrer erfindungsgemäßen programmtechnischen Einrichtung den Abfluß von Indikator aus dem Organ während des ersten Anstiegs des Eingangssignals mitberücksichtigt.

Nachfolgend wird die Erfindung anhand eines Ausführungsbeispiels unter Zuhilfenahme der zugehörigen schematischen Zeichnungen näher erläutert. Dabei zeigt

Fig. 1  schematisch die wichtigsten Komponenten der beschriebenen Ausführungsform zur Bestimmung des cerebralen Blutflusses eines Patienten und

Fig. 2  ein Schema des in der Auswerteeinheit programmtechnisch implementierten Ablaufs der Ermittlung des cerebralen Blutflusses aus dem Eingangssignal.

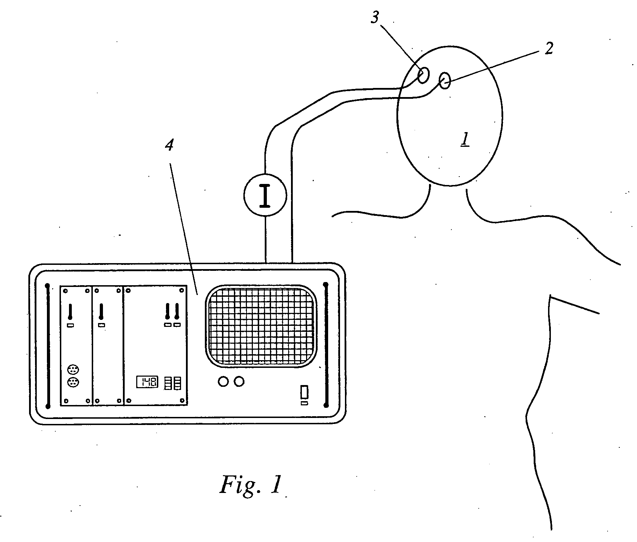

Die in Fig. 1 schematisch dargestellte erfindungsgemäße Vorrichtung dient der Bestimmung des cerebralen Blutflusses eines Patienten, beispielsweise eines Intensivpatienten in der Neurochirurgie. Am Kopf 1 des Patienten sind mittels eines (nicht dargestellten) elastischen Bandes eine erste Optode 2 und eine zweite Optode 3 in einem zueinander optimierten Abstand befestigt. Eine Strahlungsquelle (nicht dargestellt) zum Aussenden nahinfraroter Strahlung in das cerebrale Gewebe des Patienten ist entweder in der ersten Optode 2 selbst angeordnet, oder aber separat von dieser, beispielsweise in einem gemeinsamen Gehäuse mit der Auswerteeinheit 4, wobei die nahinfrarote Strahlung in letzterem Fall mittels eines Lichtleiters zur ersten Optode 2 geführt und dort emittiert wird. Die Wellenlänge der emittierten Strahlung und der verwendete Indikator müssen aufeinander abgestimmt sein. Für den üblicherweise verwendeten Indikator Indocyaningrün ist eine Wellenlänge von etwa 805 nm (jedenfalls aber im Bereich zwischen 780 und 910 nm) ideal. Die Intensität des am Ort der zweiten Optode 3 aus dem cerebralen Gewebe austretenden Anteils der infraroten Strahlung wird von dieser detektiert und an die Auswerteeinheit 4 übermittelt.

Abhängig von der Konzentration an dem Patienten injizierten Indikator im cerebralen Gewebe ist dieser Strahlungsanteil höher oder niedriger. Die auf das cerebrale Gewebe bezogene Indikatorkonzentration hängt ab von der Indikatorkonzentration im das Gewebe durchströmenden Blut sowie von der Menge des das Gewebe durchströmenden Blutes. Letztere ändert sich periodisch mit dem Herzschlag, weshalb das erhaltene Intensitätssignal einen pulsatilen, d.h. periodisch schwankenden Anteil aufweist. Dem pulsatilen Anteil des Intensitätssignals ist ein nicht-pulsatiler Anteil überlagert.

Die Indikatorkonzentration im das Gewebe durchströmenden Blut ändert sich über die Zeit dadurch, daß das aus dem cerebralen Gewebe abfließende Blut eine andere Konzentration aufweist als das zufließende Blut. Mitverantwortlich für diesen Konzentrationsunterschied ist die Verteilungskinetik des Indikators im cerebralen Gefäßsystem. Die Indikatorkonzentration in rezirkuliertem (erneut zufließendem) Blut ist zum einen bestimmt durch die Verteilungskinetik des Indikators im Gesamtkreislauf, zum anderen durch Abbau des Indikators in der Leber.

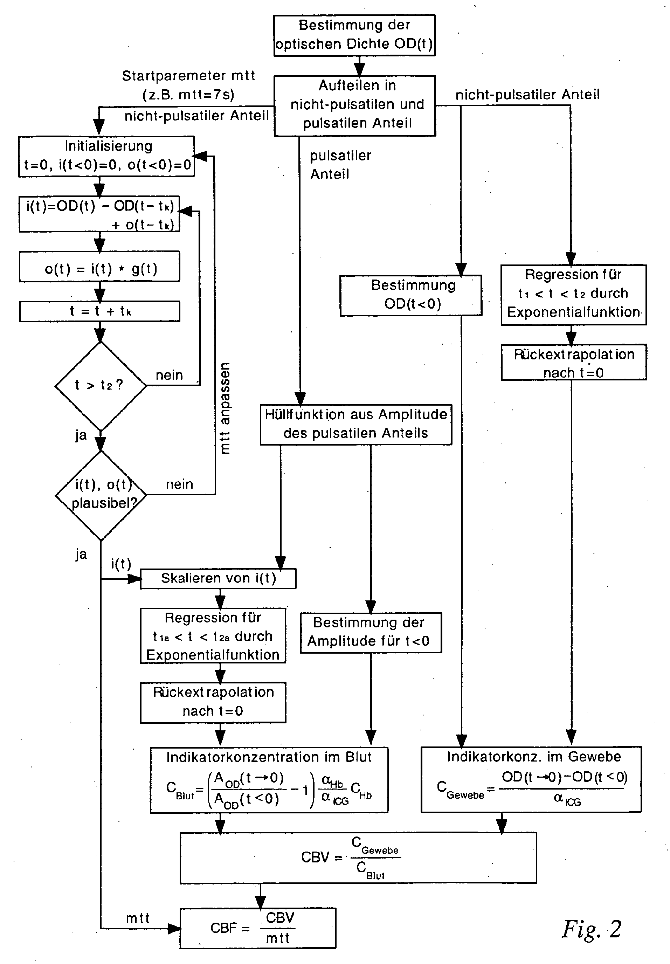

Die Auswerteeinheit 4 ist programmtechnisch zur Durchführung der in Fig. 2 in Form einer Flowchart aufgeführten und im folgenden erläuterten Auswertungsschritte eingerichtet.

Aus dem Intensitätssignal wird die optische Dichte OD als negativer dekadischer Logarithmus der Transmission gebildet. Transmission wird hierbei als Quotient aus der Intensität der detektierten nahinfraroten Strahlung und der Intensität der emittierten nahinfraroten Strahlung verstanden (und nicht im streng physikalischen Sinn, da die detektierte Strahlung gestreute und reflektierte Anteile aufweist).

Die (zeitabhängige) optische Dichte, welche im wesentlichen proportional zur Indikator-Konzentration im Gewebe ist, wird in ihren pulsatilen und ihren nicht-pulsatilen Anteil aufgeteilt.

Aus dem nicht-pulsatilen Anteil wird iterativ eine der arteriellen Eingangsfunktion des Gehirns entsprechende Zuflußfunktion i(t) bestimmt. Der Ablauf der Iteration ist im linken Ast des in Fig. 2 abgebildeten Schemas dargestellt. Als Startparameter wird eine geeignete mittlere Transitzeit mtt, beispielsweise mtt=7s, gewählt. Die mittlere Transitzeit mtt wird manchmal auch als "Durchlaufzeit" bezeichnet und ist eine charakteristische Verweilzeit, welche der Zeit entspricht, welche ein Volumenelement im Mittel benötigt, um das betrachtete System zu durchlaufen.

In einem Initialisierungsschritt werden die Zeitvariable t, sowie für den Bereich t<0 die Zuflußfunktion i(t) und die zugehörige Abflußfunktion o(t) auf null gesetzt.

Die Zuflußfunktion i(t) beschreibt den von der zufließenden Blutmenge herrührenden Anteil der Konzentrationsänderung des Indikators im cerebralen Gewebe, die Abflußfunktion o(t) den von der abfließenden Blutmenge herrührenden Anteil der Konzentrationsänderung des Indikators im cerebralen Gewebe. Der Zeitpunkt unmittelbar nach der Indikatorinjektion ist t=0.

Jeder Iterationsschritt beinhaltet die schrittweise Berechnung einer Näherung der Zuflußfunktion i(t) und einer Näherung der Abflußfunktion o(t) sowie die Berechnung einer Näherung der Transportfunktion g(t), so daß mit dem m-ten Iterationsschritt die m-te Näherung der Zuflußfunktion i(t), der Abflußfunktion o(t) und der Transportfunktion g(t) berechnet wird (m sei die Zählvariable). In einem Rechenschritt wird der Wert der Zuflußfunktion für den Zeitpunkt t gemäß der Bilanzgleichung

$$i(t) = d/dt\ (C_{Gewebe}(t)) + o(t - t_k)$$

berechnet. Darin ist $t_k$ ein konstantes, kleines Zeitintervall, so daß für $o(t-t_k)$ der Wert der Abflußfunktion zum Zeitpunkt $t-t_k$ einzusetzen ist. Der Ausdruck $d/dt\ (C_{Gewebe(t)})$ drückt die Änderung der auf das cerebrale Gewebe bezogenen Indikatorkonzentration aus.

Im Rahmen der iterativen Berechnung der Zuflußfunktion sind aufgrund einer später erfolgenden Skalierung mittels Absolutwerten des pulsatilen Signalanteils zunächst nur relative Funktionswerte erforderlich. Daher wird für die Konzentration CGewebe(t) die optische Dichte OD(t) verwendet. Ferner wird der Ausdruck d/dt (CGewebe(t)) linearisiert, so daß der obige Rechenschritt in der Form

$$i(t) = OD(t) - OD(t-t_k) + o(t-t_k)$$

implementiert ist.

Im nächsten Schritt wird für die Berechnung des Wertes der Abflußfunktion zum Zeitpunkt t das Faltungsintegral $o(t) = i(t)*g(t)$ mit der Transportfunktion $g(t)$ angesetzt. Die Transportfunktion ist nach einem der üblichen, beispielsweise bei Hoeft, A., Schorn, B., Weyland, A., Scholz, M., Buhre, W., Stepanek, E., Allen, S. J., and Sonntag, H., 1994. *Bedside assessment of intravascular volume status in patients undergoing coronary bypass surgery.* Anesthesiology 81: 76-86 veröffentlichten Ansätze gebildet und hängt von der mittleren Transitzeit mtt ab. Ein geeigneter Ansatz ist:

$$g(t) = \frac{1}{\sqrt{2 \cdot \pi \cdot \sigma \cdot t}} \cdot e^{-\frac{\left(\ln\frac{t}{mtt} + \frac{\sigma^2}{2}\right)}{2 \cdot \sigma^2}}$$

Darin ist $\sigma$ ein für das System aufgrund von Erfahrungswerten gewählter, konstanter ) Parameter (der im Grunde die Breite einer angenommenen Verweilzeitverteilung beschreibt).

In einem nächsten Schritt wird die Zeitvariable t um das Inkrement tk erhöht. Ist t kleiner als ein Endwert $t_2$, so wird die Schleife beginnend mit dem Berechnungsschritt

$$i(t) = OD(t) - OD(t-t_k) + o(t-t_k)$$

erneut durchlaufen.

Hat t den Endwert $t_2$ dagegen überschritten, so wird im nächsten Schritt geprüft, ob die erhaltenen Funktionsverläufe der Zuflußfunktion i(t) und der Abflußfunktion o(t) plausibel sind. Als Plausibilitätskriterium (d.h. Abbruchkriterium der Iteration) kann dienen, daß weder die Zuflußfunktion i(t) noch die Abflußfunktion o(t) Werte kleiner einem Schwellenwert aufweisen. Dieser Schwellenwert ist geeigneterweise größer oder gleich 0 zu wählen. Ferner kann das Plausibilitätskriterium beinhalten, daß die Zuflußfunktion i(t) als Summe einer endlichen Zahl von Funktionen darstellbar ist, welche der Form der Transportfunktion g(t) ähnlich sind.

Sind die Funktionsverläufe der Zuflußfunktion i(t) und der Abflußfunktion o(t) nicht plausibel, so wird die mittlere Transitzeit mtt durch geeignete Erhöhung oder Erniedrigung um ein Inkrement angepaßt und ein weiterer Iterationsschritt ausgeführt, welcher wiederum mit dem Initialisierungsschritt beginnt und wie dargestellt abläuft.

Sind die Funktionsverläufe der Zuflußfunktion i(t) und der Abflußfunktion o(t) dagegen plausibel, so wird die Iteration abgebrochen und mit dem erhaltenen Funktionsverlauf von i(t) sowie dem erhaltenen Wert der mittlere Transitzeit mtt weiter verfahren, wie unten beschrieben.

Ebenfalls aus dem nicht-pulsatilen Anteil des Zeitverlaufs der optischen Dichte OD wird die Indikatorkonzentration im Gewebe bestimmt, wie im rechten Ast des in Fig. 2 abgebildeten Schemas dargestellt. Hierfür wird in einem Intervall von $t_1 > 0$ bis $t_2$ der nicht-pulsatile Anteil des Zeitverlaufs der optischen Dichte OD mittels Regression durch eine Exponentialfunktion nachgebildet und diese bis zum Zeitpunkt $t=0$ rückextrapoliert. Der Zeitpunkt t2 ist so gewählt, daß eine vollständige Durchmischung des Indikators mit dem Blut gewährleistet ist, d.h. kein auf Rezirkulation beruhender Konzentrations-Peak mehr feststellbar ist. Ferner wird der Wert des nicht-pulsatilen Anteils im Bereich t<0, d.h. OD(t<0) bestimmt.

In einem nächsten Schritt erfolgt die eigentliche Berechnung der auf das Gewebe bezogenen Indikatorkonzentration unmittelbar nach der Indikatorinjektion CGewebe nach der Formel

$$CGewebe = [OD(t \rightarrow 0) - OD(t<0)] / \alpha_{ICG}$$

wobei $\alpha_{ICG}$ der Absorptionskoeffizient des Indikators ist, und $OD(t \rightarrow 0)$ dem Wert der optischen Dichte OD aus dem rückextraploierten Funktionsverlauf für t gegen 0 entspricht.

Zur Berücksichtigung des pulsatilen Anteils implementierte Auswertungsschritte sind in dem mittleren Ast der Figur 2 aufgeführt. An den pulsatilen Anteil des Zeitverlaufs der optischen Dichte OD wird eine Hüllkurve angepaßt. Ferner wird die Amplitude AOD(t<0), das heißt die Amplitude des pulsatilen Anteils der optischen Dichte für t<0 bestimmt.

Mit aus dem pulsatilen Signalanteil gewonnenen absoluten Werten aus einem Bereich, in welchem die Amplitude groß und daher weniger anfällig gegen Verrauschen ist (beispielsweise für t von 0 bis 60s), wird die zuvor iterativ ermittelte Zuflußfunktion i(t) skaliert, etwa durch Minimierung der Summe der Differenzquadrate $[i(t)- AOD(t)]^2$. Dies führt letztlich zu einer höheren Genauigkeit, denn während aus direkter Messung gewonnene Werte für größere t aufgrund geringerer Indikatorkonzentrationen zunehmend verrauscht sind, beschreibt i(t) die Abnahme der Indikatorkonzentration im Blut auch für große t gut.

Ausgehend von einem Zeitintervall von $t_{1a}>0$ bis $t_{2a}$, in welchem sich der Indikator bereits gut im System verteilt hat (d.h. mit hinreichend großen $t_{1a}$ und $t_{2a}$), wird die skalierte Zuflußfunktion i(t) bis zum Zeitpunkt t=0 rückextrapoliert (beispielsweise mittels einer durch Regression im Intervall $t_{1a}>0$ bis $t_2$ nachgebildete Exponentialfunktion).

Die für die Berechnung des cerebralen Blutflusses CBV benötigte Indikatorkonzentration im Blut $C_{Blut}$ wird nach folgender Formel berechnet, worin $A_{OD}(t\longrightarrow 0)$ dem aus der rückextrapolierten Funktion gewonnenen Wert der Amplitude der optischen Dichte für t gegen 0 entspricht (welcher gegenüber einem durch direkte Messung bestimmten Wert weniger stark von der Schärfe des ersten Signalpeaks nach Indikatorgabe abhängt):

$$C_{Blut} = \left( \frac{A_{OD}(t \to 0)}{A_{OD}(t < 0)} - 1 \right) \frac{\alpha_{Hb} C_{Hb}}{\alpha_{ICG}} \cdot$$

Darin ist $\alpha_{ICG}$ der Absorptionskoeffizient des Indikators, $\alpha_{Hb}$ der Absorptionskoeffizient des Hämoglobins und $C_{Hb}$ die Hämoglobinkonzentration im Blut.

Das cerebrale Blutvolumen CBV wird als Quotient aus der Indikatorkonzentration im Blut $C_{Blut}$ und der auf das cerebrale Gewebe bezogenen Indikatorkonzentration $C_{Gewebe}$ berechnet, d.h.:

$$CBV = C_{Gewebe} / C_{Blut} \cdot$$

Der cerebrale Blutfluß CBF wird als Quotient aus dem cerebrale Blutvolumen CBV und der mittleren Transitzeit mtt, also nach der Formel

$$CBF = CBV / mtt$$

ermittelt.

**Patentansprüche**

1. Vorrichtung zur Messung des Blutflusses in einem Organ mit Hilfe eines injizierten Indikators, aufweisend
   eine Strahlungsquelle zum Aussenden nahinfraroter Strahlung in Gewebe des Organs an einem ersten Ort,
   einen Sensor (2, 3) zum Detektieren eines aus dem Gewebe des Organs austretenden Anteils der ausgesandten nahinfraroten Strahlung an einem zweiten Ort, und
   eine Auswerteeinheit (4), welche den aus Gewebe des Organs austretenden Anteil der ausgesandten nahinfraroten Strahlung als Eingangssignal erfaßt, welches sich aus einem pulsatilen Anteil und einem nicht-pulsatilen Anteil zusammensetzt,
   **dadurch gekennzeichnet, dass** die Auswerteeinheit (4)
   programmtechnisch zur Durchführung der folgenden Auswertungsschritte eingerichtet ist:

   (a) Bestimmung der auf das Gewebe des Organs bezogenen Konzentration an injiziertem Indikator aus dem nicht-pulsatilen Anteil des Eingangssignals,

(b) iterative Ermittlung einer die Organdurchblutung charakterisierenden Zuflußfunktion i(t) durch Variation einer mittleren Transitzeit mtt bis zum Erreichen eines Abbruchkriteriums,

(c) Bestimmung der auf das Blutvolumen in dem Organ bezogenen Konzentration an injiziertem Indikator aus dem pulsatilen Anteil des Eingangssignals und der iterativ ermittelten Zuflußfunktion i(t),

(d) Berechnung des Blutvolumens in dem Organ als Quotient der auf das Gewebe des Organs bezogenen Konzentration an injiziertem Indikator und der auf das Blutvolumen in dem Organ bezogenen Konzentration an injiziertem Indikator, und

(e) Berechnung des Blutflusses in dem Organ als Quotient des Blutvolumens in dem Organ und der mittleren Transitzeit mtt bei Erreichen des Abbruchkriteriums.

2. Vorrichtung gemäß Anspruch 1, wobei eine Skalierung der Zuflußfunktion i(t) mittels aus dem pulsatilen Anteil des Eingangssignals bestimmten Werten vorgesehen ist.

3. Vorrichtung gemäß Anspruch 2, wobei die Bestimmung der auf das Blutvolumen in dem Organ bezogenen Konzentration an injiziertem Indikator eine Rückextrapolation der skalierten Zuflußfunktion i(t) bis zum Injektionszeitpunkt des Indikators umfaßt.

4. Vorrichtung gemäß einem der vorangehenden Ansprüche, wobei im Rahmen der iterativen Ermittlung der Zuflußfunktion i(t) jeder Iterationsschritt eine schrittweise näherungsweise Berechnung der Zuflußfunktion i(t) nach der Gleichung

$$i(t) = d/dt \ (C_{Gewebe}(t)) + o(t- tk)$$

und der Abflußfunktion o(t) mittels des Faltungsintegrals

$$o(t) = i(t)*g(t)$$

umfaßt,

worin d/dt ($C_{Gewebe}$(t)) ein Ausdruck ist, der die Änderung der auf das Gewebe des Organs bezogenen Konzentration an injiziertem Indikator beschreibt,
für o(t-tk) der Wert der Abflußfunktion o(t) zum Zeitpunkt t-tk einzusetzen ist, und
g(t) eine charakteristische Transportfunktion ist, in welche die mittlere Transitzeit mtt eingeht.

5. Vorrichtung gemäß einem der vorangehenden Ansprüche, wobei das Abbruchkriterium für die iterative Ermittlung der Zuflußfunktion i(t) beinhaltet, daß das Minimum der iterativ ermittelten Zuflußfunktion i(t) größer als ein Schwellenwert ist.

6. Vorrichtung gemäß Anspruch 2, wobei der Schwellenwert 0 ist.

7. Vorrichtung gemäß einem der vorangehenden Ansprüche, wobei das Abbruchkriterium für die iterative Ermittlung der Zuflußfunktion i(t) beinhaltet, daß die Zuflußfunktion i(t) als Summe einer endlichen Zahl von Funktionen darstellbar ist, welche der Form der Transportfunktion g(t) ähnlich sind.

8. Vorrichtung gemäß einem der vorangehenden Ansprüche, wobei für die Bestimmung der auf das Blutvolumen in dem Organ bezogenen Konzentration an injiziertem Indikator ein mit zunehmender Indikatorkonzentration abnehmender Absorptionskoeffizient des Indikators angesetzt wird.

9. Vorrichtung gemäß einem der vorangehenden Ansprüche, wobei die Vorrichtung für die nichtinvasive Messung des Blutflusses des Organs ausgerüstet ist und Mittel zum Einstrahlen der nahinfraroten Strahlung durch die Haut am ersten Ort und Mittel zum Auffangen des austretenden Anteils der ausgesandten nahinfraroten Strahlung durch die Haut am zweiten Ort aufweist.

10. Vorrichtung gemäß Anspruch 9, wobei die Vorrichtung ferner Mittel zur lokalen Verminderung der Durchblutung der Haut am ersten Ort und am zweiten Ort durch Aufbringen lokal erhöhten Anpreßdrucks aufweist.

11. Vorrichtung gemäß einem der vorangehenden Ansprüche, wobei die programmtechnische Einrichtung der Auswerteinheit berücksichtigt, daß das Organ das Gehirn, der Blutfluß der cerebrale Blutfluß CBF, und das Blutvolumen das cerebrale Blutvolumen CBV ist.

12. Vorrichtung gemäß einem der vorangehenden Ansprüche, wobei der Indikator Indocyaningrün ist.

**Claims**

1. Device for measuring the blood flow in an organ using an injected indicator, having a radiation source for emitting near infrared radiation into tissue of the organ at a first location, a sensor (2, 3) for detecting a proportion of the emitted near infrared radiation that exits from the tissue of the organ at a second site, and an evaluation unit (4) that detects the proportion of the emitted near infrared radiation that exits from the tissue of the organ as an input signal, which is composed of a pulsatile component and a non-pulsatile component, **characterised in that** the evaluation unit (4) is programmed to carry out the following evaluation steps:

   (a) determination of the injected indicator concentration based on the organ tissue from the non-pulsatile component of the input signal,
   (b) iterative determination of an inflow function i(t) that characterises the blood flow through the organ by variation of a mean transit time mtt until a stop criterion is reached,
   (c) determination of the injected indicator concentration based on the blood volume in the organ from the pulsatile component of the input signal and the iteratively determined inflow function i(t),
   (d) calculation of the blood volume in the organ as a quotient of the injected indicator concentration based on the organ tissue and the injected indicator concentration based on the blood volume in the organ, and
   (e) calculation of the blood flow in the organ as a quotient of the blood volume in the organ and the mean transit time mtt when the stop criterion has been reached.

2. Device according to claim 1, wherein a scaling of the inflow function i(t) is provided by means of values determined from the pulsatile component of the input signal.

3. Device according to claim 2, wherein the determination of the injected indicator concentration based on the blood volume in the organ comprises a back-extrapolation of the scaled inflow function i(t) to the injection time of the indicator.

4. Device according to any one of the preceding claims, wherein during the iterative determination of the inflow function i(t), each iteration step comprises a step-by-step calculation by approximation of the inflow function i(t) according to the equation

$$i(t) = d/dt \, (C_{tissue}(t)) + o(t - t_k)$$

and of the outflow function o(t) by means of the convolution integral

$$o(t) = i(t)*g(t)$$

wherein $d/dt \, (C_{tissue}(t))$ is a term, which describes the change in the injected indicator concentration based on the organ tissue, the value of the outflow function o(t) at the time $t-t_k$ is to be inserted for $o(t-t_k)$, and g(t) is a characteristic transport function, in which the mean transit time mtt is included.

5. Device according to any one of the preceding claims, wherein the stop criterion for the iterative determination of the inflow function i(t) includes that the minimum of the iteratively determined inflow function i(t) is greater than a threshold value.

**6.** Device according to claim 2, wherein the threshold value is 0.

**7.** Device according to any one of the preceding claims, wherein the stop criterion for the iterative determination of the inflow function i(t) includes that the inflow function i(t) can be represented as a sum of a finite number of functions which are similar to the form of the transport function g(t).

**8.** Device according to any one of the preceding claims, wherein an absorption coefficient of the indicator that decreases with an increasing indicator concentration is stated for the determination of the injected indicator concentration based on the blood volume in the organ.

**9.** Device according to any one of the preceding claims, wherein the device is equipped for the non-invasive measurement of the blood flow of the organ and has means for radiating the near infrared radiation in through the skin at the first location and means for capturing the exiting proportion of the emitted near infrared radiation through the skin at the second location.

**10.** Device according to claim 9, wherein the device also has means for local reduction of blood circulation through the skin at the first location and the second location by applying a locally increased contact pressure.

**11.** Device according to any one of the preceding claims, wherein the programming of the evaluation unit takes into account that the organ is the brain, the blood flow is cerebral blood flow CBF, and the blood volume is the cerebral blood volume CBV.

**12.** Device according to any one of the preceding claims, wherein the indicator is indocyaningreen.

## Revendications

**1.** Dispositif de mesure du flux sanguin dans un organe à l'aide d'un indicateur injecté, comprenant :

une source de rayonnement pour l'émission, à un premier endroit, d'un rayonnement proche infrarouge dans le tissu de l'organe ;
un capteur (2, 3) pour détecter une part du rayonnement proche infrarouge émis ressortant du tissu de l'organe à un second endroit ; et
une unité d'analyse (4) qui détecte une part du rayonnement proche infrarouge émis ressortant du tissu de l'organe en tant que signal d'entrée, lequel se compose d'une part pulsatile et d'une part non pulsatile ;

**caractérisé en ce que** l'unité d'analyse (4) est programmée pour exécuter les étapes d'analyse suivantes :

(a) détermination de la concentration d'indicateur injecté relative au tissu de l'organe à partir de la part non pulsatile du signal d'entrée ;
(b) détermination itérative d'une fonction d'afflux i(t) caractérisant la circulation sanguine de l'organe, par variation d'un temps de transit moyen mtt jusqu'à l'atteinte d'un critère d'interruption ;
(c) détermination de la concentration d'indicateur injecté relative au volume sanguin dans l'organe à partir de la part pulsatile du signal d'entrée et de la fonction d'afflux i(t) déterminée de manière itérative ;
(d) calcul du volume sanguin dans l'organe en tant que quotient de la concentration d'indicateur injecté relative au tissu de l'organe et de la concentration d'indicateur injecté relative au volume sanguin dans l'organe ; et
(e) calcul du flux sanguin dans l'organe en tant que quotient du volume sanguin dans l'organe et du temps de transit moyen mtt jusqu'à l'atteinte du critère d'interruption.

**2.** Dispositif selon la revendication 1, dans lequel un échelonnement de la fonction d'afflux i(t) à l'aide des valeurs déterminées à partir de la part pulsatile du signal d'entrée est prévue.

**3.** Dispositif selon la revendication 2, dans lequel la détermination de la concentration d'indicateur injecté relative au volume sanguin dans l'organe comprend une extrapolation inverse de la fonction d'afflux i(t) échelonnée jusqu'au moment d'injection de l'indicateur.

**4.** Dispositif selon l'une quelconque des revendications précédentes, dans lequel chaque étape d'itération réalisée dans le cadre de la détermination itérative de la fonction d'afflux i(t) comprend un calcul progressif par approximation de la fonction d'afflux i(t) selon l'équation

$$i(t) = d/dt\ (Ctissu(t)) + o(t\text{-}tk)$$

et de la fonction de reflux o(t) à l'aide de l'intégrale de convolution

$$o(t) = i(t)*g(t)$$

où d/dt (Ctissu(t)) décrit la modification de la concentration d'indicateur injecté relative au tissu de l'organe ;
la valeur de la fonction de reflux o(t) à l'instant t-tk doit être utilisée pour o(t-tk) ; et
g(t) est une fonction de transport caractéristique dans laquelle est inclus le temps de transit moyen mtt.

**5.** Dispositif selon l'une quelconque des revendications précédentes, dans lequel le critère d'interruption de la détermination itérative de la fonction d'afflux i(t) comprend que le minimum de la fonction d'afflux i(t) déterminée de manière itérative est supérieur à une valeur seuil.

**6.** Dispositif selon la revendication 2, dans lequel la valeur seuil est 0.

**7.** Dispositif selon l'une quelconque des revendications précédentes, dans lequel le critère d'interruption de la détermination itérative de la fonction d'afflux i(t) comprend que la fonction d'afflux i(t) peut être représentée comme la somme d'un nombre fini de fonctions prenant une forme similaire à la fonction de transport g(t).

**8.** Dispositif selon l'une quelconque des revendications précédentes, dans lequel un coefficient d'absorption de l'indicateur, diminuant lors de l'augmentation de la concentration de l'indicateur, est utilisé pour déterminer la concentration d'indicateur injecté relative au volume sanguin dans l'organe.

**9.** Dispositif selon l'une quelconque des revendications précédentes, dans lequel le dispositif est équipé de façon à permettre la mesure non invasive du flux sanguin de l'organe et présente des moyens émettant un rayonnement proche infrarouge à travers la peau à un premier endroit et des moyens captant la part de rayonnement proche infrarouge émis ressortant de la peau à un second endroit.

**10.** Dispositif selon la revendication 9, dans lequel le dispositif présente en outre des moyens permettant de réduire localement la circulation sanguine de la peau au premier et au second endroit en appliquant localement une pression accrue.

**11.** Dispositif selon l'une quelconque des revendications précédentes, dans lequel l'unité d'analyse est programmée pour prendre en compte que l'organe est le cerveau, que le flux sanguin est le flux sanguin cérébral CBF, et que le volume sanguin est le volume sanguin cérébral CBV.

**12.** Dispositif selon l'une quelconque des revendications précédentes, dans lequel l'indicateur est du vert d'indocyanine.

Fig. 1

Bestimmung der optischen Dichte OD(t)

Startparemeter mtt (z.B. mtt=7s)
nicht-pulsatiler Anteil

Aufteilen in nicht-pulsatilen und pulsatilen Anteil

nicht-pulsatiler Anteil

pulsatiler Anteil

Initialisierung
$t=0$, $i(t<0)=0$, $o(t<0)=0$

$i(t)=OD(t) - OD(t-t_k) + o(t-t_k)$

$o(t) = i(t) * g(t)$

$t = t + t_k$

$t > t_2$?    nein

ja

mtt anpassen

$i(t)$, $o(t)$ plausibel?    nein

ja

$i(t)$

Bestimmung OD(t<0)

Regression für $t_1 < t < t_2$ durch Exponentialfunktion

Rückextrapolation nach $t=0$

Hüllfunktion aus Amplitude des pulsatilen Anteils

Skalieren von $i(t)$

Regression für $t_{1a} < t < t_{2a}$ durch Exponentialfunktion

Bestimmung der Amplitude für $t<0$

Rückextrapolation nach $t=0$

Indikatorkonzentration im Blut
$$C_{Blut} = \left( \frac{A_{OD}(t \to 0)}{A_{OD}(t<0)} - 1 \right) \frac{\alpha_{Hb}}{\alpha_{ICG}} C_{Hb}$$

Indikatorkonz. im Gewebe
$$C_{Gewebe} = \frac{OD(t \to 0) - OD(t<0)}{\alpha_{ICG}}$$

$$CBV = \frac{C_{Gewebe}}{C_{Blut}}$$

mtt

$$CBF = \frac{CBV}{mtt}$$

*Fig. 2*